## Europäisches Patentamt

## European Patent Office

## Office européen des brevets

(11) Veröffentlichungsnummer: **0 076 987**
A2

# EUROPÄISCHE PATENTANMELDUNG

(21) Anmeldenummer: **82109097.4**

(22) Anmeldetag: **01.10.82**

(51) Int. Cl.³: **A 61 K 7/48, A 61 K 35/72**

(30) Priorität: **01.10.81 DE 3139093**

(43) Veröffentlichungstag der Anmeldung: **20.04.83** Patentblatt **83/16**

(84) Benannte Vertragsstaaten: **AT CH FR LI NL**

(71) Anmelder: **Dr. Babor GmbH & Co., Neuenhofstrasse 180, D-5100 Aachen (DE)**

(72) Erfinder: **Schimanski, Dieter, Dr., Kircheichstrasse 162, D-5120 Herzogenrath (DE)**

(74) Vertreter: **Kressin, Horst-R., Dr. et al, DIEHL & KRESSIN Flüggenstrasse 17, D-8000 München 19 (DE)**

(54) **Kosmetisches Mittel.**

(57) Die Erfindung betrifft kosmetische Mittel, die neben üblichen kosmetischen Grundstoffen einen Gehalt an nativem Hefezellinhalt aufweisen. Der Hefezellinhalt wird durch mechanisches oder chemisches Aufbrechen der Hefezellwände und durch Abtrennen der Zellwände und gegebenenfalls der Zellkernmembranen unter schonenden Bedingungen hergestellt. Die Erfindung betrifft des weiteren Hefezellinhalt-Fraktionen, die angereichert sind an Hefezellkernfraktionen, die bei der Verwendung in kosmetischen Mitteln eine besonders hohe hautpflegende Wirkung aufweisen. Der native Hefezellinhalt ist der Zellinhalt ohne äußere Zellwände oder der Zellinhalt ohne äußere Zellwände und wenigstens teilweise abgetrennten Zellkernmembranen.

Kosmetisches Mittel

Beschreibung

Die Erfindung betrifft ein kosmetisches Mittel, das gekennzeichnet ist durch einen Gehalt an Hefezellinhalt-Fraktionen.

Hefe ist eine einzellige Pflanze der Klasse Ascomycetes, die in der Natur weit verbreitet ist. Diese Pflanze gehört zu der Pilzfamilie der Saccharomycetaceae. Hefe wird aufgrund des Gehalts an Aminosäuren, Mineralstoffen, Spurenelementen, Vitaminen und anderen Wirkstoffen, z.B. in Form von Bierhefeflocken und -tabletten oder in getrockneter Form, als gesundheitsförderndes Mittel eingenommen. Die Hefekuren sollen gegen unreine und fette Haut wirken.

Es ist auch bekannt, medizinische Hefe als Zusatz zu Gesichtspackungen zur Behandlung der Gesichtshaut zu verwenden (Karl Rothemann, Das große Rezeptbuch der Haut- und Körperpflegemittel, 4. Auflage, S. 180).

In der FR-PS 870 386 ist ein hefehaltiges Kosmetikum beschrieben, das zur Verbesserung der Hautatmung einsetzbar sein soll. Hierbei wird Hefe mit siedendem Äthanol mehrere Stunden behandelt, und aus dem alkoholischen Filtrat wird mit Barytlauge ein nicht näher definiertes Produkt ausgefällt, das in eine Creme oder Seife eingearbeitet wird. Durch die Behandlung mit Alkohol in der Wärme wird ein großer Teil des Zellinhalts der Hefezellen zerstört bzw. in eine inaktive, denaturierte Form überführt, wodurch die gesundheitsfördernde Wirkung der Hefe erheblich beeinträchtigt wird.

0076987

Der Erfindung liegt daher die Aufgabe zugrunde, ein Kosmetikum auf Basis eines Hefematerials zur Verfügung zu stellen, das eine hautpflegende und hautregenerierende Wirkung aufweist. Außerdem soll das kosmetische Mittel die Feuchtigkeit der Haut regulieren, die Haut tonisieren, glätten und die Durchblutung der Haut fördern.

Die Erfindung wird gelöst durch ein kosmetisches Mittel, das neben üblichen kosmetischen Grundstoffen einen Gehalt an nativen Hefezellinhalt-Fraktionen aufweist.

Die erfindungsgemäß eingesetzten nativen Hefezellinhalt-Fraktionen bestehen vorzugsweise aus Hefezellkernfraktionen, die aus Hefezellen durch Zerstörung der Zellwände und der Zellmembranen erhalten werden. Die Hefezellkernfraktionen sind im wesentlichen frei von Hefezellmembranen. Die Hefezellkernfraktionen enthalten vorzugsweise Desoxyribonucleinsäuren und den Repair-Komplex der Hefezellkerne. Die üblichen kosmetischen Grundstoffe, die zusammen mit der Hefezellinhalt-Präparation verwendet werden sind z.B. Glycerin, Alkohol, Polyäthylenglykol, Parfümöle, Konservierungsmittel, Carboxymethylcellulose, Carboxyvinylmischpolymerisat, Bienenwachs, Maiskeimöl, Cholesterin, Paraffinöl, Magnesiumsulfat, Decyloleat, Salbengrundlage usw.

Das erfindungsgemäße kosmetische Mittel besitzt eine sehr gute hautglättende und feuchtigkeitsregulierende Eigenschaft. Hautrauhigkeiten werden bei Behandlung mit dem erfindungsgemäßen kosmetischen Mittel, insbesondere bei Behandlung mit Lotionen, kurzfristig und anhaltend beseitigt. Die Haut erhält kurz nach der Behandlung mit den erfindungsgemäßen Mitteln ein vorteilhaft samtiges Aussehen. Neben der hautglättenden Sofortwirkung besitzen insbesondere die kosmetischen Mittel, die die Hefezellkernfraktionen enthalten, eine wirksame hautregenerierende Eigenschaft.

- 3 -

0076987

Die Hefenucleinsäuren greifen direkt in das Zellgeschehen der Hautzellen ein und stellen somit eine wirksame Aktivsubstanz gegen die Zellalterung dar.

Die Hefenucleinsäuren sind reich an hydrophilen Gruppen, die stark hydratisierend wirken. Die Hefenucleinsäuren sind wahrscheinlich mit für die Bildung des feuchtigkeitsbewahrenden Films auf der Epidermis verantwortlich.

Die enzymatischen Systeme der Hefezelle, wie die Invertasen, bauen abgestorbene Hornhautteilchen ab, die das dermatologische Gleichgewicht und das Funktionieren der Talgdrüsen stören. Die erfindungsgemäß eingesetzten Hefezellinhalt-Fraktionen bewirken daher eine Verbesserung der Hauthygiene.

Der bei der Hefezellzerschlagung anfallende native Hefezellinhalt-Extrakt enthält in Abhängigkeit vom jeweiligen Ausgangsmaterial einen unterschiedlichen Proteingehalt. Im allgemeinen liegt der Gehalt bei etwa 0,5 bis 2 %. Der Hefezellinhalt-Extrakt wird daher zur Erleichterung der Anwendung durch Verdünnen mit der verwendeten Suspensionsflüssigkeit, z.B. Trispuffer, auf 1 % Protein standardisiert.

Die erfindungsgemäßen kosmetischen Mittel enthalten insbesondere mehr als 1 Gew.-% Zellinhalt-Extrakt bzw. Fraktion (standardisiert auf 1 % Protein).

Die verwendete Menge liegt zweckmäßig bei 2 bis 20 Gew.-%, vorzugsweise 5 bis 10 Gew.-% Hefezellinhalt-Extrakt (standardisiert auf 1 % Protein), bezogen auf das Gesamtgewicht des kosmetischen Mittels. Höhere Gehalte können zwar verwendet werden, sie sind aus wirtschaftlichen Überlegungen jedoch nicht empfehlenswert.

Die erfindungsgemäßen nativen Zellinhaltfraktionen sind insbesondere nach dem folgenden Verfahren herstellbar.

Als Ausgangsmaterial sind die verschiedensten handelsüblichen Hefen einsetzbar, insbesondere die Saccharomyces-Hefearten, wie Bäckerhefe, Bierhefe, Weinhefe, Milchhefe und Torula. Die Hefe wird in destilliertem Wasser, Kochsalzlösung, die gegebenenfalls einen Zusatz von Saccharose enthält, oder in Trispuffer-Lösung (Tris-(hydroxymethyl)-amino-methan-Puffer) suspendiert und dann unter Kühlung gemahlen. Das Aufschließen der Hefezellen kann z.B. durch Zermahlen in Zellmühlen oder Zellbomben vorgenommen werden. Es sind z.B. Schwingmühlen geeignet, in denen die die Hefesuspension enthaltende Flasche eingespannt ist. In der Flasche sind kleine Kugeln mit einem Durchmesser von etwa 0,2 bis 0,5 mm enthalten, die die Zerschlagung der Zellen bewirken. Die Schwingfrequenz beträgt zwischen 2000 bis 4000 Perioden/min. Die Behandlungsdauer der Hefezellsuspension beträgt im allgemeinen einige Sekunden bis wenige Minuten. Bei 4000 Perioden/min ist der Zerschlagungsgrad der Hefezellen nach etwa 20 bis 120 sec fast vollständig. Bei 4000 Perioden/min werden z.B. nach 120 sec nur noch 0,05 % unzerstörte Hefezellen ermittelt. Während des Mahlvorganges wird die die Suspension enthaltende Flasche von außen z.B. mit Kohlensäure gekühlt, so daß die Temperatur in der gekühlten Hefesuspension nicht oder nur minimal ansteigt. Der bevorzugte Bereich während des Zellaufschlusses liegt bei etwa 0 bis 5°C. Das Homogenisat wird durch Zentrifugieren und/oder durch Filtration von den unzerstörten Hefezellen, den Zellwänden und gegebenenfalls dem Zellkernmembranmaterial getrennt. Der Aufschluß kann aber auch durch Druckeinwirkung, Ultraschallbearbeitung oder chemische Weise vorgenommen werden.

Die Bestimmung der durch den Zellaufschluß frei gewordenen nativen Zellinhaltsstoffe erfolgt durch die Proteinbestimmung, z.B. mit Hilfe der Biurethreaktion nach Bücher. Die Messung erfolgt vorzugsweise photometrisch gegen einen Blindwert oder durch UV-Messung. Die Berechnung erfolgt z.B. nach den folgenden Gesetzmäßigkeiten:

mg Protein im Cuvettenansatz = Extinktion x 17

oder

mg Protein/ml Lösung = $1,45 \cdot \text{Ext.}_{280} - 0,74 \cdot \text{Ext.}_{260}$

(280 = Absorptionsmaximum der Proteine)
(260 = Absorptionsmaximum der DNS)

Die Enzymaktivität des nativen Hefezellinhalts kann durch die Alkohol-dehydrogenase-Aktivität bestimmt werden (vgl. Biochemische Zeitschrift, Bd. 329 (1957), S. 334).

Die überraschende Wirkung der erfindungsgemäßen Hefezellinhalt-Fraktionen wird darauf zurückgeführt, daß es sich hierbei um native Zellinhaltsstoffe handelt, die aufgrund der schonenden Behandlung die volle Wirksamkeit besitzen, wobei sich die Wirkung der Zellinhaltsstoffe auf die Haut nicht nur addiert, sondern offenbar durch synergistisch wirkende Stoffe des Zellkerns erhöht. Beim mechanischen Aufbrechen der Hefezellwände und/oder der Zellkernmembranen werden die Eiweiße nicht denaturiert, die Vitamine nicht beeinträchtigt und die Desoxyribonucleinsäuren (DNS) und der Repair-Komplex der Hefezellkerne nicht zerstört. Derartige native Hefezellinhalt-Fraktionen sind bisher nicht in der kosmetischen Industrie verwendet worden. Wie der Literaturstelle Dr. J.S.Jellinek, Kosmetologie, S. 105, zu entnehmen ist, haben Hefen bisher für den Kosmetiker wenig Bedeutung gehabt.

Es wird der gesamte komplexe native Hefezellinhalt als Zusatz zu kosmetischen Mitteln gemäß der Erfindung verwendet. Wenn das erfindungsgemäße kosmetische Mittel jedoch eine besonders hohe hautregenerierende Eigenschaft besitzen soll, ist darauf zu achten, daß die Hefezellkernmembranen möglichst weitgehend beim Aufschluß der

0076987

Hefezellen zerstört werden und die native Hefezellinhalt-Fraktion möglichst viel Hefezellkerninhalt-Fraktionen enthält. Der native Hefezellinhalt kann aber auch einer weiteren Trennung unterzogen werden, um die Hefezellkernfraktionen anzureichern. Die Hefezellkernfraktion ist besonders reich an den Hefenucleinsäuren und dem Repair-Komplex.

Der Wirkungsmechanismus des Repair-Komplexes der Hefezellkerne ist etwa wie folgt zu verstehen:

Durch starke UV-Lichteinstrahlung entstehen Schäden an der DNS. Diese Schäden sind durch ein körpereigenes Abwehrsystem reparierbar. Es wird angenommen, daß die durch die UV-Strahlung entstandene schadhafte Stelle der DNS durch den Einbau neuer Bausteine repariert wird und so der intakte Strang der DNS wieder geschlossen wird. Es wird angenommen, daß der Repair-Komplex in der Lage ist, den Einbau der neuen Bausteine zu beschleunigen. Der hier aus den Hefezellkernen hergestellte Wirkstoff entfaltet seine Wirkung nicht im Bindegewebe, sondern an den unteren Zellschichten der Epidermis. Die Ursache für seine Wirkung ist bisher nicht bekannt. Es wurde weiterhin festgestellt, daß die Hefezellkernfraktion, die den Repair-Komplex enthält, in ihrer Reparaturkapazität durch Wärmeeinwirkung beeinträchtigt wird. Die Sonneneinstrahlung fördert somit die Hautschäden, die durch UV-Strahlung verursacht werden. Der neue kosmetische Wirkstoff erhöht also die Reparaturfähigkeit der Hautzellen, wie durch Versuche ermittelt wurde.

Die Erfindung wird anhand der nachfolgenden Beispiele näher erläutert:

0076987

Beispiel 1

Es wurden 810 g Bäckerhefe in 750 g Trispuffer (Tris-(hydroxymethyl)-amino-methan-Puffer) mit einem pH-Wert von 8,0 suspendiert. Die Suspension wurde dann in einer Zellmühle mittels Glaskugeln mit einem Durchmesser von 0,2 bis 0,5 mm bei 0 bis 5°C gemahlen. Der Durchfluß wurde so eingestellt, daß 90 % der Hefezellen aufgerieben wurden. Als Zellmühle wurde die Dynomill KDL verwendet. Anschließend wurde die Suspension von Zellwandfragmenten und nicht zerstörten Hefezellen durch Abzentrifugieren mit 6000 g über 10 Min. getrennt. Der Überstand wurde mit Trispuffer-Lösung verdünnt im Verhältnis von ca. 1:1, so daß eine standardisierte Lösung mit 1 % Protein entstand. Die standardisierte 1 %ige Hefeproteinfraktion wurde mit Konservierungsmitteln versetzt und dann im Kühlschrank bei 4°C für die Weiterverarbeitung aufbewahrt.

Die so hergestellte Hefezellinhalt-Präparation A wurde zu einem kosmetischen Fluid bzw. einer kosmetischen Lotion der folgenden Zusammensetzung verarbeitet:

10000 Gew.-% Hefezellinhalt-Präparation A (standardisiert 1 % Protein)
 7500 Gew.-% Glycerin oder Sorbit
 7500 Gew.-% Polyoxyäthyliertes Triglycerid
  250 Gew.-% Carboxyvinylmischpolymerisat
   60 Gew.-% Konservierungsmittel
 7500 Gew.-% Propanol-1
10000 Gew.-% Polyäthylenglykol
   30 Gew.-% Melissenöl
ad 100000 Gew.-% destilliertes Wasser

Beispiel 2

Es wurden 20 g Bäckerhefe 48 Std. unter Vakuum getrocknet und dann in 4 %iger Kochsalzlösung, die 2 % Saccharose enthielt, suspendiert. Nach einer Lagerung von 2 Std. bei 28°C wurde die Hefe auf einer Filternutsche abgesaugt und mit destilliertem Wasser gewaschen. Die zurückbleibende Hefe wurde in destilliertem Wasser in einem Verhältnis von 100 g Naßhefe zu 450 g Wasser unter Zusatz von Enzymglucoronidas / Arylsufatase (100000 Fishman-Einheiten / 1.000.000 Roy-Einheiten) in einer Zellbombe bei etwa 0 bis 5°C   1 Std. unter 200 atü Stickstoff gerührt. Nach dem Dekomprimieren wurden die Hefezellkerne durch Saccharosezusatz abzentrifugiert und in der Zellmühle  nach Bühler mittels Glasperlen eines Durchmessers von 0,2 bis 0,5 mm gemahlen. Nach der Sedimentation der Glasperlen wurde die Hefezellinhalt-Fraktion B erhalten.

Die so hergestellte Hefezellinhalt-Fraktion B wurde in eine kosmetische Creme mit der folgenden Zusammensetzung eingearbeitet:

```
10000 Gew.-% Salbengrundlage
 4000 Gew.-% Decyloleat
 4000 Gew.-% Dodecyloleat
 6000 Gew.-% Maiskeimöl
 1000 Gew.-% Cholesterin
 1000 Gew.-% Bienenwachs
 6000 Gew.-% Isopropylmyristat
 1000 Gew.-% polyoxyäthyliertes Triglycerid
 4000 Gew.-% Paraffinöl
 3000 Gew.-% Sorbit
  200 Gew.-% Magnesiumsulfat
   50 Gew.-% Konservierungsmittel
  200 Gew.-% Panthenol
  100 Gew.-% Parfümierungsmittel
10000 Gew.-% Hefezellinhalt-Fraktion B
             (standardisiert 1 % Protein)
ad 100000 Gew.-% destilliertes Wasser
```

**Beispiel 3**

Anwendungsbeispiel

Die Hautlotion gemäß Beispiel 1 wurde so eingestellt, daß der Gehalt an Hefepräparation A (standardisiert auf 1 % Protein) auf 5 Gew.-% bzw. 10 Gew.-%, bezogen auf das Gesamtgewicht der Lotion, betrug, und dann wurde die Wirkung dieser beiden Lotionen auf rauhe, schuppige und trockene Haut untersucht, indem man diese Lotionen auf mehrere Stellen entsprechend vorbereiteter Hände von Testpersonen auftrug. Die Hände der Testpersonen wurden vor der Behandlung in einer Kalk-Zement-Mischung behandelt, um die Hände rauh, schuppig und trocken zu machen. Bei der Behandlung mit den erfindungsgemäßen Testlotionen normalisierte sich das äußere Erscheinungsbild der entsprechend behandelten Hautstellen sofort. Die Wirkung wurde von den Testpersonen als angenehm entspannend beschrieben. Die Durchblutung der oberen Hautschicht wurde gefördert. Der Tonus der Haut wurde verbessert, und die Hautoberfläche erhielt eine angenehme Geschmeidigkeit. Bei der Lotion mit 5 Gew.-% der erfindungsgemäßen Hefepräparation A tritt die Hautrauhigkeit nach 4 bis 6 Stunden in stark abgeschwächter Form hervor. Bei der Lotion mit einem Gehalt von 10 Gew.-% der Hefepräparation A tritt die Hautrauhigkeit auch nach 6 h und mehr nicht mehr auf.

Beispiel 4

Vergleichsversuch

Die Hautlotion gemäß Beispiel 1, die die Hefepräparation A enthält, wurde so eingestellt, daß die Lotion 10 Gew.-% der Hefepräparation A enthielt. Dann wurde diese Lotion auf der Haut von 24 Testpersonen hinsichtlich der Wirkung auf die Haut untersucht. Als Vergleichsversuch wurde die gleiche Hautlotion hergestellt, wobei jedoch anstelle der erfindungsgemäßen Hefepräparation A die gleiche Menge unbehandelte Bäckerhefe verwendet wurde.

Die erfindungsgemäße Hautlotion bzw. das erfindungsgemäße Hautfluid wurde von allen Testpersonen als signifikant in ihrer Wirkung auf die Haut erkannt. Diese Lotion wirkte hautentspannend, glättend, und die Lotion erhöhte den Feuchtigkeitsgehalt der oberen Hautschicht merklich. Eine entsprechende Hautwirkung wurde von den Testpersonen für das Vergleichsfluid, das lediglich unbehandelte Bäckerhefe enthielt, nicht festgestellt.

## DIEHL & KRESSIN

PATENTANWÄLTE · EUROPEAN PATENT ATTORNEYS

Zugelassen bei den deutschen und europäischen Patentbehörden

Flüggenstraße 17 · D-8000 München 19

0076987

1. Oktober 1982

Ba 1930-US

Dr. Babor GmbH & Co.

Neuenhofstr. 180

5100 Aachen

Kosmetisches Mittel

Patentansprüche

1. Kosmetisches Mittel mit einem Gehalt an Hefe, d a d u r c h   g e k e n n z e i c h n e t , daß es neben üblichen kosmetischen Grundstoffen einen Gehalt an nativem Hefezellinhalt aufweist.

0076987

2. Kosmetisches Mittel nach Anspruch 1, dadurch gekennzeichnet, daß der native Hefezellinhalt im wesentlichen frei von Hefezellwänden ist.

3. Kosmetisches Mittel nach Anspruch 1, dadurch gekennzeichnet, daß der native Hefezellinhalt im wesentlichen frei von Hefezellwänden und Hefezellkernmembranen ist.

4. Kosmetisches Mittel nach einem der Ansprüche 1 bis 3, dadurch gekennzeichnet, daß der native Hefezellinhalt im wesentlichen aus einer Hefezellkerninhalt-Fraktion enthaltend den Repair-Komplex der Hefezellkerne, besteht.

5. Kosmetisches Mittel nach Anspruch 1, dadurch gekennzeichnet, daß der native Hefezellinhalt aus Hefezellen durch Abtrennung der Zellwände und gegebenenfalls der Zellkernmembranen herstellbar ist.

6. Kosmetisches Mittel nach Anspruch 1, dadurch gekennzeichnet, daß es die native Hefezellinhalt-Fraktion, Glycerin, polyoxyäthyliertes Triglycerid, Carboxyvinyl-mischpolymerisat, Konservierungsmittel, Propanol, Poly-äthylenglykol, destilliertes Wasser und/oder ein Parfümierungsmittel enthält.

7. Kosmetisches Mittel nach einem der vorhergehenden Ansprüche, dadurch gekennzeichnet, daß der Gehalt an Hefezellinhalt-Fraktion (standardisiert auf 1 % Protein) mehr als 1 Gew.-%, bezogen auf das Gesamtgewicht des kosmetischen Mittels, beträgt.

8. Kosmetisches Mittel nach Anspruch 7, dadurch gekennzeichnet, daß der Gehalt an Hefezellinhalt-Fraktion (standardisiert auf 1% Protein) 2 bis 20 Gew.-%, insbesondere 5 bis 10 Gew.-%, bezogen auf das Gesamtgewicht des kosmetischen Mittels, beträgt.

0076987

9. Verfahren zur Herstellung des kosmetischen Mittels nach einem der Ansprüche 1 bis 6, dadurch gekennzeichnet, daß man Hefezellen unter Kühlung aufschließt, die unzerstörten Hefezellen, die Hefezellwände und gegebenenfalls die Hefezellkernmembranen abtrennt und den nativen Hefezellinhalt mit üblichen kosmetischen Grundstoffen zu einem kosmetischen Mittel vermischt.

10. Verfahren nach Anspruch 9, dadurch gekennzeichnet, daß der Aufschluß der Hefezellen durch Zermahlen, Druckeinwirkung, Ultraschallbearbeitung oder chemische Bearbeitung der Hefe erfolgt.

11. Verwendung des kosmetischen Mittels nach einem der Ansprüche 1 bis 8 in Hautpflegecremes, Sonnenschutzpräparaten, Gesichtslotionen, Gesichtswassern, Körperlotionen, Rasierpräparaten, Make-up-Präparaten oder Badezusätzen.